# EUROPÄISCHE PATENTSCHRIFT

(11) **EP 4 583 818 B1**
(45) Veröffentlichungstag und Bekanntmachung des Hinweises auf die Patenterteilung: **11.11.2026**
(21) Anmeldenummer: 23764845.6
(22) Anmeldetag: 29.08.2023
(51) Int. Cl.: A61F 2/16, A61F 2/00

(54) **INJEKTOR FÜR EINE INTRAOKULARLINSE**
INJECTOR FOR AN INTRAOCULAR LENS
INJECTEUR POUR UNE LENTILLE INTRAOCULAIRE

(30) Priorität: 09.09.2022 DE 102022122961
(43) Veröffentlichungstag der Anmeldung: 16.07.2025
(73) Patentinhaber: Carl Zeiss Meditec AG, 07745 Jena (DE)
(72) Erfinder: BADUR, Thorben, 73447 Oberkochen (DE); SCHARSICH, Christina, 73447 Oberkochen (DE); MASCH, Jennifer-Magdalena, 73447 Oberkochen (DE); MOEIN, Hadi, 73447 Oberkochen (DE)
(74) Vertreter: PATERIS PartmbB
(86) Internationale Anmeldenummer: PCT/EP2023/073709
(87) Internationale Veröffentlichungsnummer: WO 2024/052175

(56) Entgegenhaltungen:
- EP-A1- 2 340 786
- DE-B3- 102021 116 615
- US-A1- 2020 197 158

## Beschreibung

Die Erfindung betrifft einen Injektor für eine Intraokularlinse und eine Verpackung mit dem Injektor.

Bei einer Kataraktbehandlung eines Auges wird herkömmlich nur ein kleiner Schnitt in die Hornhaut des Auges eingebracht, der so groß ist, dass eine Spitze eines Injektors durch den Schnitt in das Auge eingeführt werden kann. Nachdem der Schnitt in die Hornhaut eingebracht wurde, wird die Linse des Auges üblicherweise mittels Phakoemulsifikation zerkleinert und anschließend aus dem Kapselsack des Auges abgesaugt. Danach wird eine Intraokularlinse mittels des Injektors in das Auge eingesetzt. Der Injektor wird herkömmlich in einer sterilen Verpackung geliefert, wobei die Verpackung frühestens kurz vor der Kataraktbehandlung geöffnet wird. Die Verpackung ist danach zu entsorgen. Dabei fällt jedoch nachteilig eine große Menge an Abfall an. DE 10 2021 116 615 B3 beschreibt einen Injektor mit einem ersten Kolben und einem zweiten Kolben.

Aufgabe der Erfindung ist es daher, einen Injektor für ein Einführen einer Intraokularlinse in den Kapselsack eines Auges und eine Verpackung mit dem Injektor zu schaffen, bei denen nur eine geringe Menge an Abfall anfällt.

Der erfindungsgemäße Injektor für ein Einführen einer Intraokularlinse in den Kapselsack eines Auges weist einen Injektorkörper und einen teleskopierenden Kolben auf. Der Injektorkörper weist eine Spitze mit einer Spitzenöffnung, ein der Spitzenöffnung abgewandtes proximales Ende und eine Einführrichtung auf, die von dem proximalen Ende zu der Spitzenöffnung orientiert ist. Der teleskopierende Kolben ist längsverlagerbar in dem Injektorkörper angeordnet und weist mehrere Teilkolben auf. Zudem hat der teleskopierende Kolben einen Einfahrzustand, in dem der teleskopierende Kolben eingefahren ist, und einen Ausfahrzustand, in dem der teleskopierende Kolben ausgefahren ist und in dem alle Paare der Teilkolben, wobei jedes der Paare aus zwei benachbarten der Teilkolben besteht, starr in der Einführrichtung miteinander gekoppelt sind. Einer der Teilkolben ist ein erster Teilkolben, der in dem Ausfahrzustand das in der Einführrichtung angeordnete Längsende des teleskopierenden Kolbens bildet, und ein anderer der Teilkolben ist ein letzter Teilkolben, der in dem Ausfahrzustand das entgegen der Einführrichtung angeordnete Längsende des teleskopierenden Kolben bildet. Der Injektor ist eingerichtet, dass der teleskopierende Kolben durch ein Verlagern des letzten Teilkolbens entgegen der Einführrichtung von dem Einfahrzustand in den Ausfahrzustand bringbar ist, wobei der Injektor lediglich die von dem ersten Teilkolben verschiedenen der Teilkolben aufweist, um ein erstes Verlagern durchzuführen, das die von dem ersten Teilkolben verschiedenen der Teilkolben relativ zu dem ersten Teilkolben durchführen, wobei das erste Verlagern erforderlich ist, um den teleskopierenden Kolben von dem Einfahrzustand in den Ausfahrzustand zu bringen.

Der Injektor hat in dem Einfahrzustand des teleskopierenden Kolbens einer kürzeren Erstreckung als in dem Ausfahrzustand des teleskopierenden Kolbens. Indem der Injektor in dem Einfahrzustand des teleskopierenden Kolbens in einer Verpackung angeordnet wird, kann diese Verpackung mit einer kürzeren Länge in der Einführrichtung und mit einem kleineren Volumen als bei einer herkömmlichen Verpackung ausgeführt werden, in der ein herkömmlicher Injektor ohne den teleskopierenden Kolben angeordnet ist. Dadurch fällt vorteilhaft nur eine geringe Menge an Abfall an.

Zumindest eines der Paare der Teilkolben bildet bevorzugt einen Einrastmechanismus für das jeweilige Paar, wobei mittels des Einrastmechanismus die Teilkolben des Paares in dem Ausfahrzustand starr in der Einführrichtung miteinander gekoppelt sind. Es ist auch denkbar, dass mehrere der Paare oder alle der Paare jeweils einen Einrastmechanismus für das jeweilige Paar bilden. Es ist besonders bevorzugt, dass der Einrastmechanismus von einem Vorsprung, der von einem der Teilkolben des Paares gebildet ist und der gegen den anderen der Teilkolben des Paares vorgespannt ist, und einer Aussparung gebildet ist, die in den anderen der Teilkolben des Paares eingebracht ist, wobei in dem Ausfahrzustand der Vorsprung in die Aussparung eingreift und außerhalb des Ausfahrzustandes der Vorsprung vollständig außerhalb der Aussparung angeordnet ist.

Es ist bevorzugt, dass der Injektor für zumindest eines der Paare jeweils einen Sperrkörper aufweist, der verlagerbar und/oder verschwenkbar angeordnet ist und somit von einer ersten Position, in der einer der Teilkolben des Paares relativ in der Einführrichtung zu dem anderen der Teilkolben des Paares verlagerbar ist, in eine zweite Position verlagerbar und/oder verschwenkbar ist, in der der eine Teilkolben des Paares an den Sperrkörper anstößt, wenn der eine Teilkolben des Paares in der Einführrichtung relativ zu dem anderen der Teilkolben des Paares verlagert, und somit eingerichtet ist, anschließend den anderen der Teilkolben des Paares in der Einführrichtung mitzunehmen. Dabei ist besonders bevorzugt, dass der Injektorkörper ein Injektorkörperdurchgangsloch aufweist, via das der Sperrkörper zugänglich ist. Dadurch kann der Sperrkörper beispielsweise von einer Hand und/oder mittels eines Werkzeugs von außerhalb des Injektorkörpers von der ersten Position in die zweite Position verlagert werden.

In dem Einfahrzustand sind die von dem ersten Teilkolben verschiedenen der Teilkolben bevorzugt innerhalb des ersten Teilkolbens angeordnet. Alternativ ist denkbar, dass in dem Einfahrzustand die von dem letzten Teilkolben verschiedenen der Teilkolben innerhalb des letzten Teilkolbens angeordnet sind.

Es ist bevorzugt, dass der Injektorkörper einen Anschlag aufweist, gegen den der erste Teilkolben eingerichtet ist anzuschlagen und der somit ein Längsverlagern des ersten Teilkolbens entgegen der Einführrichtung begrenzt. Dadurch verbleibt der erste Teilkolben an dem Anschlag, wenn während des ersten Verlagerns, insbesondere durch ein Verlagern des letzten Teilkolbens entgegen der Einführrichtung, der teleskopierende Kolben von dem Einfahrzustand in den Ausfahrzustand gebracht wird. Dabei ist besonders bevorzugt, dass die von dem ersten Teilkolben verschiedenen der Teilkolben nicht eingerichtet sind, gegen den Anschlag anzuschlagen.

Der Injektor weist bevorzugt ein Vorspannmittel auf, das eingerichtet ist, den teleskopierenden Kolben von dem Einfahrzustand in den Ausfahrzustand zu befördern. Bei dem Vorspannmittel kann es sich beispielsweise um eine Druckfeder und/oder ein Gefäß handeln, das mit einem Gas, dessen Druck höher als der Umgebungsdruck des Injektors ist, beaufschlagt ist. Bei dem Gas kann es sich beispielsweise um Luft handeln. Es ist besonders bevorzugt, dass der Injektor eine Arretierung aufweist, die eingerichtet ist, den letzten Kolben lösbar in dem Einfahrzustand zu halten. Durch ein Lösen der Arretierung kann der teleskopierende Kolben durch das Vorspannmittel von dem Einfahrzustand in den Ausfahrzustand gebracht werden.

Es ist bevorzugt, dass der erste Kolben eingerichtet ist, die Intraokularlinse zu kontaktieren und durch ein Verlagern des ersten Kolbens in der Einführrichtung aus der Spitzenöffnung heraus zu befördern.

Es ist bevorzugt, dass der Injektor die Intraokularlinse aufweist, die in der Einführrichtung zwischen dem ersten Teilkolben und der Spitzenöffnung angeordnet ist. Alternativ ist bevorzugt, dass der Injektorkörper eine Aufnahme aufweist, die eingerichtet ist, eine Kartusche, in der die Intraokularlinse angeordnet ist, aufzunehmen.

In der erfindungsgemäßen Verpackung ist der Injektor angeordnet, wobei der teleskopierende Kolben sich in dem Einfahrzustand befindet.

Ein nicht zu der Erfindung gehörendes Verfahren zum Bedienen eines Injektors weist die Schritte auf: - Bereitstellen des Injektors für ein Einführen einer Intraokularlinse in den Kapselsack eines Auges, wobei der Injektor einen teleskopierenden Kolben und einen Injektorkörper aufweist, der eine Spitze mit einer Spitzenöffnung, ein der Spitzenöffnung abgewandtes proximales Ende und eine Einführrichtung aufweist, die von dem proximalen Ende zu der Spitzenöffnung orientiert ist, wobei der teleskopierende Kolben längsverlagerbar in dem Injektorkörper angeordnet ist, mehrere Teilkolben aufweist sowie einen Einfahrzustand, in dem der teleskopierende Kolben eingefahren ist, und einen Ausfahrzustand hat, in dem der teleskopierende Kolben ausgefahren ist und in dem alle Paare der Teilkolben, wobei jedes der Paare aus zwei benachbarten der Teilkolben besteht, starr in der Einführrichtung miteinander gekoppelt sind, wobei einer der Teilkolben ein erster Teilkolben ist, der in dem Ausfahrzustand das in der Einführrichtung angeordnete Längsende des teleskopierenden Kolbens bildet, und ein anderer der Teilkolben ein letzter Teilkolben ist, der in dem Ausfahrzustand das entgegen der Einführrichtung angeordnete Längsende des teleskopierenden Kolben bildet; - Bringen des teleskopierenden Kolbens von dem Einfahrzustand in den Ausfahrzustand, indem der letzte Teilkolben entgegen der Einführrichtung verlagert wird. Der teleskopierende Kolben kann beispielsweise automatisch von dem Einfahrzustand in den Ausfahrzustand gelangen, wenn der Injektor aus der Verpackung herausgenommen wird, insbesondere in dem Fall, dass der Injektor das Vorspannmittel aufweist.

Es ist bevorzugt, dass der Injektor in einer Verpackung angeordnet ist, der teleskopierende Kolben sich in dem Einfahrzustand befindet und das Verfahren den Schritt aufweist: - Öffnen der Verpackung und Herausnehmen des Injektors aus der Verpackung. Dabei ist denkbar, dass das Bringen des teleskopierenden Kolbens von dem Einfahrzustand in den Ausfahrzustand nach dem Öffnen der Verpackung erfolgt.

Bevorzugt weist das Verfahren den Schritt auf: - Durchführen eines zweiten Verlagerns, indem der sich in dem Ausfahrzustand befindliche teleskopierende Kolben in der Einführrichtung verlagert wird. Dadurch kann die Intraokularlinse via die Spitzenöffnung aus dem Injektor heraus verlagert werden.

Im Folgenden wird anhand der beigefügten schematischen Zeichnungen die Erfindung näher erläutert. Es zeigen
Figur 1 eine perspektivische Ansicht einer ersten Ausführungsform eines erfindungsgemäßen Injektors zu drei verschiedenen Zeitpunkten,
Figur 2 eine perspektivische Ansicht einer zweiten Ausführungsform des Injektors zu zwei verschiedenen Zeitpunkten und
Figur 3 eine Draufsicht auf eine Verpackung mit einer dritten Ausführungsform des Injektors.

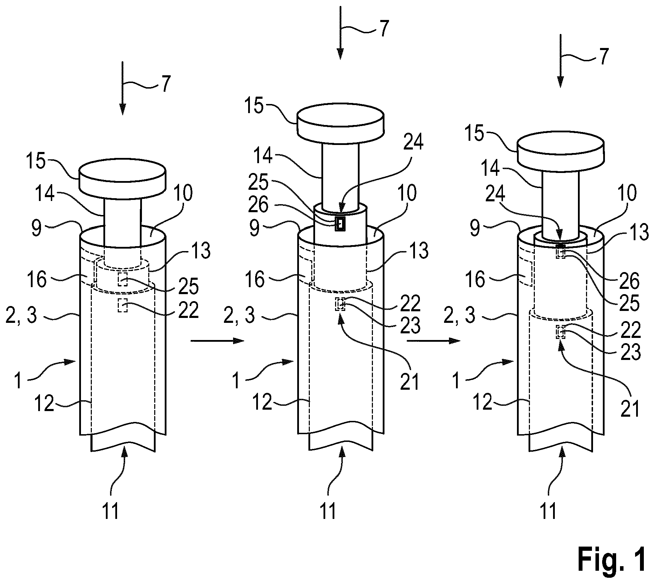

Wie es aus Figuren 1 bis 3 ersichtlich ist, weist ein Injektor 1 für ein Einführen einer Intraokularlinse 60 in den Kapselsack eines Auges einen Injektorkörper 2 und einen teleskopierenden Kolben 11 auf. Der Injektorkörper 2 weist eine Spitze 5 mit einer Spitzenöffnung 8, ein der Spitzenöffnung 8 abgewandtes proximales Ende 9 und eine Einführrichtung 7 auf, die von dem proximalen Ende 9 zu der Spitzenöffnung 8 orientiert ist. Der teleskopierende Kolben 11 ist längsverlagerbar in dem Injektorkörper 2 angeordnet und weist mehrere Teilkolben 12, 13, 14 auf. Der teleskopierende Kolben 11 hat einen Einfahrzustand, in dem der teleskopierende Kolben 11 eingefahren ist, und einen Ausfahrzustand, in dem der teleskopierende Kolben 11 ausgefahren ist und in dem alle Paare der Teilkolben 12, 13, 14, wobei jedes der Paare aus zwei benachbarten der Teilkolben 12, 13, 14 besteht, starr in der Einführrichtung 7 miteinander gekoppelt sind. Einer der Teilkolben **12, 13,** 14 ist ein erster Teilkolben **12,** der in dem Ausfahrzustand das in der Einführrichtung 7 angeordnete Längsende des teleskopierenden Kolbens **11** bildet. Ein anderer der Teilkolben **12, 13,** 14 ist ein letzter Teilkolben **14,** der in dem Ausfahrzustand das entgegen der Einführrichtung 7 angeordnete Längsende des teleskopierenden Kolben **11** bildet. Der Injektor 1 ist eingerichtet, dass der teleskopierende Kolben **11** durch ein Verlagern des letzten Teilkolbens 14 entgegen der Einführrichtung 7 von dem Einfahrzustand in den Ausfahrzustand bringbar ist, wobei der Injektor 1 lediglich die von dem ersten Teilkolben 12 verschiedenen der Teilkolben **12, 13,** 14 aufweist, um ein erstes Verlagern durchzuführen, das die von dem ersten Teilkolben 12 verschiedenen der Teilkolben **12, 13,** 14 relativ zu dem ersten Teilkolben 12 durchführen, wobei das erste Verlagern erforderlich ist, um den teleskopierenden Kolben **11** von dem Einfahrzustand in den Ausfahrzustand zu bringen. Es ist demnach zusätzlich zu den von dem ersten Teilkolben 12 verschiedenen der Teilkolben **12, 13,** 14 kein weiteres Bauteil in dem Injektor 1 vorgesehen, mittels dem das erste Verlagern durchgeführt werden kann. Insbesondere ist kein weiteres Bauteil vorgesehen, mittels dem der erste Teilkolben 12 in die Einführrichtung verlagerbar ist.

Es ist denkbar, dass der Injektorkörper 2 ein Innengewinde aufweist und der teleskopierende Kolben 21 ein Außengewinde aufweist, das mit dem Innengewinde in Eingriff steht. Dadurch kann der teleskopierende Kolben **11** in dem Ausfahrzustand durch ein Rotieren des teleskopierenden Kolbens **11** um seine Längsachse längsverlagert werden. Alternativ ist denkbar, dass der teleskopierende Kolben **11** durch ein reines Drücken des teleskopierenden Kolbens **11** in Richtung seiner Längsachse längsverlagert werden kann.

Dadurch, dass jedes der Paare starr in der Einführrichtung miteinander gekoppelt ist, können alle die Teilkolben **12, 13,** 14 gemeinsam in der Einführrichtung 7 verlagert werden, beispielsweise indem der letzte Teilkolben 12, 13, 14 in die Einführrichtung 7 verlagert wird, insbesondere gedrückt oder rotiert wird.

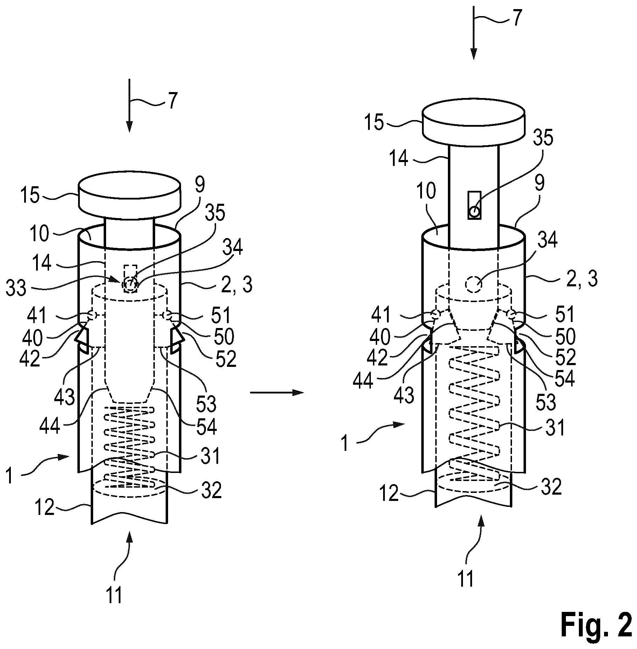

Figuren 1 und 2 zeigen, dass in dem Einfahrzustand die von dem ersten Teilkolben 12 verschiedenen der Teilkolben 12, 13, 14 innerhalb des ersten Teilkolbens 12 angeordnet sein können.

Der teleskopierende Kolben in dem Einfahrzustand ist in Figur 1 in der linken Zeichnung und in Figur 2 in der linken Zeichnung dargestellt. Der teleskopierende Kolben in dem Ausfahrzustand ist in Figur 1 in der mittleren Zeichnung und in der rechten Zeichnung und in Figur 2 in der rechten Zeichnung dargestellt. Figuren 1 und 2 zeigen, dass der Injektorkörper 2 eine Injektorkörperöffnung 10 aufweisen kann, die der Spitzenöffnung 8 abgewandt angeordnet ist und via die der teleskopierende Kolben 11 in dem Ausfahrzustand nach außerhalb des Injektorkörpers 2 ragt. Es ist auch denkbar, dass der teleskopierende Kolben 11 auch in dem Einfahrzustand aus der Injektorkörperöffnung 10 ragt. Durch das erste Verlagern wird der teleskopierende Kolben 11 in den Ausfahrzustand gebracht, indem der letzte Kolben 14 entgegen der Einführrichtung 7 verlagert wird. Dies ist in Figur 1 in der mittleren Zeichnung und in Figur 2 in der rechten Zeichnung dargestellt. Der Injektorkörper 2 kann einen Anschlag 16 aufweisen (siehe Figur 1), gegen den der erste Teilkolben 12 eingerichtet ist anzuschlagen und der somit ein Längsverlagern des ersten Teilkolbens 12 entgegen der Einführrichtung 7 bei dem ersten Verlagern begrenzt. Die von dem ersten Teilkolben 12 verschiedenen der Teilkolben 12, 13, 14 sind eingerichtet, nicht gegen den Anschlag 16 anzuschlagen. Durch das erste Verlagern verlängert sich die Erstreckung des Injektors 1 in der Einführrichtung 7. Nach dem ersten Verlagern kann, beispielsweise indem der letzte Teilkolben 14 in die Einführrichtung 7 gedrückt wird, ein zweites Verlagern durchgeführt werden, bei dem der teleskopierende Kolben 11 in der Einführrichtung 7 verlagert wird, vergleiche die rechte Zeichnung in Figur **1****.** Dadurch ist es möglich, die Intraokularlinse 60 via die Spitzenöffnung 8 aus dem Injektor 1 zu befördern. Dabei ist es denkbar, dass der erste Teilkolben 12 ein Kissen 17 aufweist, das das in der Einführrichtung 7 angeordnete Längsende des ersten Teilkolbens 12 bildet und eingerichtet ist, gequetscht zu werden, wenn es in die Spitze 5 gelangt. Insbesondere kann der erste Teilkolben 12 einen Schaft 18 aufweisen, an dem das Kissen 17 befestigt ist und der von dem ersten Teilkolben 12 vorsteht.

Die Anzahl der Paare beträgt eins weniger als die Anzahl der Teilkolben **12, 13, 14.** Die Anzahl der Teilkolben **12, 13, 14** kann beispielsweise drei betragen, vergleiche Figur **1****,** oder zwei betragen, vergleiche Figur **2****.** Es ist jedoch auch eine Anzahl der Teilkolben **12, 13,** 14 denkbar, die größer als drei ist. In Figur 1 ist dementsprechend die Anzahl der Paare zwei, wobei der erste Teilkolben 12 und ein zweiter Teilkolben 13 ein erstes Paar bilden und der zweite Teilkolben 13 und der letzte Teilkolben 14 ein zweites Paar bilden.

Figur 1 zeigt, dass zumindest eines der Paare einen Einrastmechanismus **21,** 24 für das jeweilige Paar bilden kann, wobei mittels des Einrastmechanismus **21,** 24 die Teilkolben **12, 13,** 14 des Paares in dem Ausfahrzustand starr in der Einführrichtung 7 miteinander gekoppelt sind. In dem Beispiel gemäß Figur 1 ist die Anzahl der Teilkolben drei und somit die Anzahl der Paare zwei. Um alle die Paare starr in der Einführrichtung miteinander zu koppeln, können daher ein erster Einrastmechanismus **21,** um den ersten Teilkolben 12 und einen zweiten Teilkolben 13 starr in der Einführrichtung 7 miteinander zu koppeln, und ein zweiter Einrastmechanismus 22 vorgesehen sein, um den zweiten Teilkolben 13 und den letzten Teilkolben 14 starr in der Einführrichtung 7 miteinander zu koppeln. Der erste Einrastmechanismus 21 kann beispielsweise von einem Vorsprung 23 (vergleiche die mittlere Zeichnung und die rechte Zeichnung in Figur **1****),** der von zweiten Teilkolben 13 gebildet ist und der gegen den ersten Teilkolben 12 vorgespannt ist, und einer Aussparung 22 gebildet sein, die in den ersten Teilkolben 12 eingebracht ist, wobei in dem Ausfahrzustand der Vorsprung 23 in die Aussparung 22 eingreift. In analoger Weise kann der zweite Einrastmechanismus 24 beispielsweise von einem Vorsprung 26 (vergleiche die mittlere Zeichnung und die rechte Zeichnung in Figur **1****),** der von letzten Teilkolben 14 gebildet ist und der gegen den zweiten Teilkolben 13 vorgespannt ist, und einer Aussparung 25 gebildet sein, die in den zweiten Teilkolben 13 eingebracht ist, wobei in dem Ausfahrzustand der Vorsprung 26 in die Aussparung 25 eingreift. Außerhalb des Ausfahrzustands kann der Vorsprung 23 außerhalb der Aussparung 22 und/oder der Vorsprung 26 außerhalb der Aussparung 25 angeordnet sein.

Figur 2 zeigt, dass der Injektor 1 für zumindest eines der Paare einen Sperrkörper 40 aufweisen kann, der verlagerbar und/oder verschwenkbar angeordnet ist und somit von einer ersten Position, in der einer der Teilkolben **12, 13,** 14 des Paares relativ in der Einführrichtung 7 zu dem anderen der Teilkolben **12, 13,** 14 des Paares verlagerbar ist, in eine zweite Position verlagerbar und/oder verschwenkbar ist, in der der eine Teilkolben **12, 13,** 14 des Paares an den Sperrkörper 40 anstößt, wenn der eine Teilkolben **12, 13,** 14 des Paares in der Einführrichtung 7 relativ zu dem anderen der Teilkolben **12, 13,** 14 des Paares verlagert, und somit eingerichtet ist, anschließend den anderen der Teilkolben **12, 13,** 14 des Paares in der Einführrichtung 7 mitzunehmen. Der andere der Teilkolben **12, 13,** 14 kann ein Lager 41 aufweisen, an dem der Sperrkörper 40 verschwenkbar gelagert ist. Der Injektorkörper 2 kann ein Injektorkörperdurchgangsloch 42 aufweisen, via das der Sperrkörper 40 zugänglich ist. Zudem kann der andere der Teilkolben **12, 13,** 14 ein Kolbendurchgangsloch 43 aufweisen, via das der Sperrkörper 40 sich erstreckt. Bei dem einem der Teilkolben **12, 13,** 14 kann es sich beispielsweise um den letzten Teilkolben 14 handeln und bei dem anderen der Teilkolben **12, 13,** 14 kann es sich beispielsweise um den ersten Teilkolben 12 handeln, vergleiche Figur **2****.**

Es ist zudem denkbar, dass das Paar einen weiteren Sperrkörper 50 aufweist, der verlagerbar und/oder schwenkbar angeordnet ist und somit von einer ersten Position, in der der eine Teilkolben **12, 13,** 14 des Paares relativ in der Einführrichtung 7 zu dem anderen der Teilkolben **12, 13,** 14 des Paares verlagerbar ist, in eine zweite Position verlagerbar und/oder verschwenkbar ist, in der der eine Teilkolben **12, 13,** 14 des Paares an den weiteren Sperrkörper 50 anstößt, wenn der eine Teilkolben **12, 13,** 14 des Paares in der Einführrichtung 7 relativ zu dem anderen der Teilkolben **12, 13,** 14 des Paares verlagert, und somit eingerichtet ist, anschließend den anderen der Teilkolben **12, 13,** 14 des Paares in der Einführrichtung 7 mitzunehmen. Der andere der Teilkolben **12, 13,** 14 kann ein weiteres Lager 42 aufweisen, an dem der weitere Sperrkörper 50 verschwenkbar gelagert ist. Der Injektorkörper 2 kann ein weiteres Injektorkörperdurchgangsloch 52 aufweisen, via das der weitere Sperrkörper 50 zugänglich ist. Zudem kann der andere der Teilkolben **12, 13,** 14 ein Kolbendurchgangsloch 53 aufweisen, via das der weitere Sperrkörper 50 sich erstreckt.

Der eine der Teilkolben **12, 13,** 14 kann eine erste Kolbenflanke 44 aufweisen, die eingerichtet ist, den Sperrköper 40 zu kontaktieren. Zudem kann der eine der Teilkolben **12, 13,** 14 eine weitere Kolbenflanke 54 aufweisen, die eingerichtet ist, den weiteren Sperrkörper 50 zu kontaktieren. Die erste Kolbenflanke 44 und die weitere Kolbenflanke 54 können geneigt zu der Einführrichtung 7 angeordnet sein und insbesondere beabstandet voneinander angeordnet sein.

Wie es aus Figur 2 ersichtlich ist, kann der Injektor 1 ein Vorspannmittel 31 aufweisen, das eingerichtet ist, den teleskopierenden Kolben **11** von dem Einfahrzustand in den Ausfahrzustand zu befördern. Bei dem Vorspannmittel 31 kann es sich beispielsweise um eine Druckfeder (vergleiche Figur 2) und/oder um einen Ballon, der mit einem unter Überdruck stehenden Gas gefüllt ist, handeln. Der Injektorkörper 2 kann eine Abstützfläche 32 aufweisen, die das Vorspannmittel 31 entgegen der Einführrichtung 7 abstützt. Das Vorspannmittel 31 kann an seiner der Abstützfläche 32 abgewandten Seite den letzten Kolben 14 kontaktieren. Der Injektor 1 kann eine Arretierung 33 aufweisen, die eingerichtet ist, den letzten Kolben 14 lösbar in dem Einfahrzustand zu halten. Beispielsweise kann die Arretierung 33 von einem Durchgangsloch 34, das in dem Injektorkörper 2 angeordnet ist, und einem Stift 35 gebildet sein, das verlagerbar an dem letzten Kolben 14 befestigt ist. Indem der Stift 35 in dem Durchgangsloch 34 angeordnet ist, ist der teleskopierende Kolben 11 in dem Einfahrzustand gehalten. Indem der Stift 35 aus dem Durchgangsloch 34 entfernt wird, kann das Vorspannmittel 31 den teleskopierenden Kolben 11 in den Ausfahrzustand verlagern.

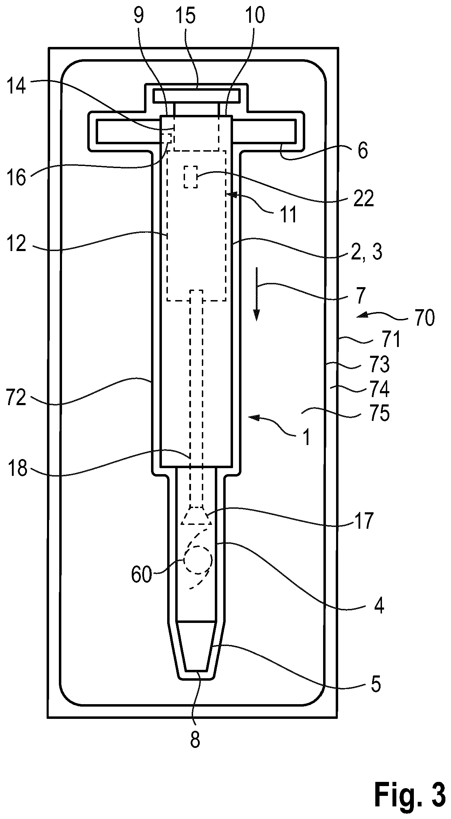

Figur 3 zeigt, dass der Injektor 1 in einer Verpackung 70 angeordnet sein kann und sich der teleskopierende Kolben 11 dabei in dem Einfahrzustand befinden kann. Die Verpackung 70 kann eine Basis 71 aufweisen, die insbesondere eine Vertiefung 72 aufweisen kann, in die der Injektor 1 angeordnet ist. Die Verpackung 70 kann zudem einen Deckel 73 aufweisen, der zusammen mit der Basis 71 einen Innenraum 75 der Verpackung 70 begrenzt, wobei der Injektor 1 in dem Innenraum 75 angeordnet ist. Die Basis 71 und der Deckel 73 können sich in einer vollständig umlaufenden Dichtfläche 74 kontaktieren, so dass der Innenraum 75 hermetisch abgedichtet ist. Der Deckel 73 kann transparent sein, wie es in Figur 3 beispielsweise dargestellt ist.

Der Injektorkörper 2 kann einen Zylinder 3 und eine Ladekammer 4 aufweisen, wobei die Ladekammer 4 in der Einführrichtung 7 zwischen dem Zylinder 3 und der Spitze 5 angeordnet ist. Der Injektor 1 kann die Intraokularlinse 60 aufweisen, die in der Ladekammer 4 sowie in der Einführrichtung 7 zwischen dem ersten Kolben 12 und der Spitzenöffnung 8 angeordnet ist. Alternativ ist denkbar, dass die Intraokularlinse 60 nicht in dem Injektor 1 angeordnet ist, sondern erst in die Ladekammer 4 eingesetzt wird, nachdem die Verpackung 70 geöffnet wurde und der Injektor 1 aus der Verpackung 70 entnommen wurde.

Zudem kann der Injektorkörper 2 einen Griff 6 aufweisen, der von dem Zylinder 3 absteht. Der letzte Teilkolben 14 kann eine an seinem entgegen der Einführrichtung 7 angeordneten Längsende angeordnete Daumenauflage 15 aufweisen, die eine Verdickung des letzten Teilkolbens 14 ist. Dabei ist denkbar, dass die Daumenauflage 15 so dimensioniert ist, dass die Daumenauflage 15 bei dem zweiten Verlagern gegen das proximale Ende 9 anstößt.

### Bezugszeichenliste

- 1: Injektor
- 2: Injektorkörper
- 3: Zylinder
- 4: Ladekammer
- 5: Spitze
- 6: Griff
- 7: Einführrichtung
- 8: Spitzenöffnung
- 9: proximales Ende
- 10: Injektorkörperöffnung
- 11: telekopierender Kolben
- 12: erster Teilkolben
- 13: zweiter Teilkolben
- 14: letzter Teilkolben
- 15: Daumenauflage
- 16: Anschlag
- 17: Kissen
- 18: Schaft
- 21: erster Einrastmechanismus
- 22: Aussparung
- 23: Vorsprung
- 24: zweiter Einrastmechanismus
- 25: Aussparung
- 26: Vorsprung
- 31: Vorspannmittel
- 32: Abstützfläche
- 33: Arretierung
- 34: Durchgangsloch
- 35: Stift
- 40: Sperrkörper
- 41: Lager
- 42: Injektorkörperdurchgangsloch
- 43: Kolbendurchgangsloch
- 44: Kolbenflanke
- 50: weiterer Sperrkörper
- 51: weiteres Lager
- 52: weiteres Injektorkörperdurchgangsloch
- 53: weiteres Kolbendurchgangsloch
- 54: weitere Kolbenflanke
- 60: Intraokularlinse
- 70: Verpackung
- 71: Basis
- 72: Vertiefung
- 73: Deckel
- 74: Dichtfläche
- 75: Innenraum

## Patentansprüche

1. Injektor für ein Einführen einer Intraokularlinse (60) in den Kapselsack eines Auges, mit einem Injektorkörper (2), der eine Spitze (5) mit einer Spitzenöffnung (8), ein der Spitzenöffnung (8) abgewandtes proximales Ende (9) und eine Einführrichtung (7) aufweist, die von dem proximalen Ende (9) zu der Spitzenöffnung (8) orientiert ist, und einem teleskopierenden Kolben (11), der längsverlagerbar in dem Injektorkörper (2) angeordnet ist, mehrere Teilkolben (12, 13, 14) aufweist sowie einen Einfahrzustand, in dem der teleskopierende Kolben (11) eingefahren ist, und einen Ausfahrzustand hat, in dem der teleskopierende Kolben (11) ausgefahren ist und in dem alle Paare der Teilkolben (12, 13, 14), wobei jedes der Paare aus zwei benachbarten der Teilkolben (12, 13, 14) besteht, starr in der Einführrichtung (7) miteinander gekoppelt sind, wobei einer der Teilkolben (12, 13, 14) ein erster Teilkolben (12) ist, der in dem Ausfahrzustand das in der Einführrichtung (7) angeordnete Längsende des teleskopierenden Kolbens (11) bildet, und ein anderer der Teilkolben (12, 13, 14) ein letzter Teilkolben (14) ist, der in dem Ausfahrzustand das entgegen der Einführrichtung (7) angeordnete Längsende des teleskopierenden Kolben (11) bildet, wobei der Injektor (1) eingerichtet ist, dass der teleskopierende Kolben (11) durch ein Verlagern des letzten Teilkolbens (14) entgegen der Einführrichtung (7) von dem Einfahrzustand in den Ausfahrzustand bringbar ist, **dadurch gekennzeichnet, dass** der Injektor (1) lediglich die von dem ersten Teilkolben (12) verschiedenen der Teilkolben (13, 14) aufweist, um ein erstes Verlagern durchzuführen, das die von dem ersten Teilkolben (12) verschiedenen der Teilkolben (13, 14) relativ zu dem ersten Teilkolben (12) durchführen, wobei das erste Verlagern erforderlich ist, um den teleskopierenden Kolben (11) von dem Einfahrzustand in den Ausfahrzustand zu bringen.

2. Injektor gemäß Anspruch 1, wobei zumindest eines der Paare einen Einrastmechanismus (21, 24) für das jeweilige Paar bildet, wobei mittels des Einrastmechanismus (21, 24) die Teilkolben (12, 13, 14) des Paares in dem Ausfahrzustand starr in der Einführrichtung (7) miteinander gekoppelt sind.

3. Injektor gemäß Anspruch 2, wobei der Einrastmechanismus (21, 24) von einem Vorsprung (23, 26), der von einem der Teilkolben (12, 13, 14) des Paares gebildet ist und der gegen den anderen der Teilkolben (12, 13, 14) des Paares vorgespannt ist, und einer Aussparung (22, 25) gebildet ist, die in den anderen der Teilkolben (12, 13, 14) des Paares eingebracht ist, wobei in dem Ausfahrzustand der Vorsprung (23, 26) in die Aussparung (22, 25) eingreift.

4. Injektor gemäß einem der Ansprüche 1 bis 3, wobei der Injektor (1) für zumindest eines der Paare einen Sperrkörper (40) aufweist, der verlagerbar und/oder verschwenkbar angeordnet ist und somit von einer ersten Position, in der einer der Teilkolben (12, 13, 14) des Paares relativ in der Einführrichtung (7) zu dem anderen der Teilkolben (12, 13, 14) des Paares verlagerbar ist, in eine zweite Position verlagerbar und/oder verschwenkbar ist, in der der eine Teilkolben (12, 13, 14) des Paares an den Sperrkörper (40) anstößt, wenn der eine Teilkolben (12, 13, 14) des Paares in der Einführrichtung (7) relativ zu dem anderen der Teilkolben (12, 13, 14) des Paares verlagert, und somit eingerichtet ist, anschließend den anderen der Teilkolben (12, 13, 14) des Paares in der Einführrichtung (7) mitzunehmen.

5. Injektor gemäß einem der Ansprüche 1 bis 4, wobei in dem Einfahrzustand die von dem ersten Teilkolben (12) verschiedenen der Teilkolben (13, 14) innerhalb des ersten Teilkolbens (12) angeordnet sind.

6. Injektor gemäß einem der Ansprüche 1 bis 5, wobei der Injektorkörper (2) einen Anschlag (16) aufweist, gegen den der erste Teilkolben (12) eingerichtet ist anzuschlagen und der somit ein Längsverlagern des ersten Teilkolbens (12) entgegen der Einführrichtung (7) begrenzt.

7. Injektor gemäß einem der Ansprüche 1 bis 6, wobei der Injektor (1) ein Vorspannmittel (31) aufweist, das eingerichtet ist, den teleskopierenden Kolben (11) von dem Einfahrzustand in den Ausfahrzustand zu befördern.

8. Injektor gemäß einem der Ansprüche 1 bis 7, wobei der Injektor (1) die Intraokularlinse (60) aufweist, die in der Einführrichtung (7) zwischen dem ersten Teilkolben (12) und der Spitzenöffnung (8) angeordnet ist.

9. Verpackung mit dem Injektor (1) gemäß einem der Ansprüche 1 bis 8, wobei der Injektor (1) in der Verpackung (70) angeordnet ist und der teleskopierende Kolben (11) sich in dem Einfahrzustand befindet.

## Claims

1. Injector for inserting an intraocular lens (60) into the capsular bag of an eye, comprising an injector body (2) which has a tip (5) having a tip opening (8), a proximal end (9) remote from the tip opening (8) and an insertion direction (7) oriented from the proximal end (9) to the tip opening (8), and comprising a telescopic plunger (11) which is mounted longitudinally displaceably in the injector body (2), has multiple partial plungers (12, 13, 14) and has a retracted state in which the telescopic plunger (11) is retracted and an extended state in which the telescopic plunger (11) is extended, and in which all pairs of the partial plungers (12, 13, 14), each pair consisting of two adjacent partial plungers (12, 13, 14), are rigidly coupled to one another in the insertion direction (7), wherein one of the partial plungers (12, 13, 14) is a first partial plunger (12) which, in the extended state, forms the longitudinal end of the telescopic plunger (11) arranged in the insertion direction (7) and another of the partial plungers (12, 13, 14) is a last partial plunger (14) which, in the extended state, forms the longitudinal end of the telescopic plunger (11) arranged counter to the insertion direction (7), wherein the injector (1) is configured to be able to bring the telescopic plunger (11) from the retracted state into the extended state by displacing the last partial plunger (14) counter to the insertion direction (7), **characterized in that** the injector (1) has merely the partial plungers (13, 14) different from the first partial plunger (12) for carrying out a first displacement that the partial plungers (13, 14) different from the first partial plunger (12) carry out relative to the first partial plunger (12), wherein the first displacement is required for bringing the telescopic plunger (11) from the retracted state into the extended state.

2. Injector according to Claim 1, wherein at least one of the pairs forms a latching mechanism (21, 24) for the respective pair, wherein the partial plungers (12, 13, 14) of the pair are rigidly coupled to one another in the insertion direction (7) in the extended state by means of the latching mechanism (21, 24).

3. Injector according to Claim 2, wherein the latching mechanism (21, 24) is formed by a projection (23, 26) which is formed by one of the partial plungers (12, 13, 14) of the pair and which is biased against the other of the partial plungers (12, 13, 14) of the pair and a recess (22, 25) which is introduced into the other of the partial plungers (12, 13, 14) of the pair, wherein the projection (23, 26) engages in the recess (22, 25) in the extended state.

4. Injector according to any of Claims 1 to 3, wherein the injector (1) has for at least one of the pairs a blocking body (40) which is mounted displaceably and/or pivotably and is thus displaceable and/or pivotable from a first position in which one of the partial plungers (12, 13, 14) of the pair is displaceable relative to the other of the partial plungers (12, 13, 14) of the pair in the insertion direction (7) into a second position in which the one partial plunger (12, 13, 14) of the pair knocks against the blocking body (40) when the one partial plunger (12, 13, 14) of the pair is displaced relative to the other of the partial plungers (12, 13, 14) of the pair in the insertion direction (7), and is thus configured to subsequently carry along the other of the partial plungers (12, 13, 14) of the pair in the insertion direction (7).

5. Injector according to any of Claims 1 to 4, wherein the partial plungers (13, 14) different from the first partial plunger (12) are arranged within the first partial plunger (12) in the retracted state.

6. Injector according to any of Claims 1 to 5, wherein the injector body (2) has a stop (16) against which the first partial plunger (12) is configured to strike and which thus limits a longitudinal displacement of the first partial plunger (12) counter to the insertion direction (7).

7. Injector according to any of Claims 1 to 6, wherein the injector (1) has a biasing means (31) configured to convey the telescopic plunger (11) from the retracted state into the extended state.

8. Injector according to any of Claims 1 to 7, wherein the injector (1) has the intraocular lens (60) arranged in the insertion direction (7) between the first partial plunger (12) and the tip opening (8).

9. Package containing the injector (1) according to any of Claims 1 to 8, wherein the injector (1) is arranged in the package (70) and the telescopic plunger (11) is in the retracted state.

## Revendications

1. Injecteur destiné à insérer une lentille intraoculaire (60) dans le sac capsulaire d'un œil, avec un corps (2) d'injecteur, qui comporte une pointe (5) avec une ouverture (8) de pointe, une extrémité proximale (9) opposée à l'ouverture (8) de pointe et une direction d'insertion (7), qui est orientée depuis l'extrémité proximale (9) vers l'ouverture (8) de pointe, et avec un piston télescopique (11), qui est disposé de manière à pouvoir se déplacer longitudinalement dans le corps (2) d'injecteur, qui comporte plusieurs sous-pistons (12, 13, 14) et présente un état de retrait, dans lequel le piston télescopique (11) est rentré, et un état déployé, dans lequel le piston télescopique (11) est déployé, et dans lequel toutes les paires des sous-pistons (12, 13, 14), chacune des paires étant constituée de deux sous-pistons adjacents des sous-pistons (12, 13, 14), sont couplées rigidement les unes aux autres dans la direction d'insertion (7), l'un des sous-pistons (12, 13, 14) étant un premier sous-piston (12), qui forme dans l'état déployé l'extrémité longitudinale du piston télescopique (11) disposée dans la direction d'insertion (7), et un autre des sous-pistons (12, 13, 14) étant un dernier sous-piston (14) qui forme, dans l'état déployé, l'extrémité longitudinale du piston télescopique (11) disposée dans le sens inverse de la direction d'insertion (7), l'injecteur (1) étant mis au point pour que le piston télescopique (11) puisse être amené depuis l'état rentré dans l'état déployé par un déplacement du dernier sous-piston (14) dans le sens inverse de la direction d'insertion (7), **caractérisé en ce que** l'injecteur (1) ne comporte que les sous-pistons, différents du premier sous-piston (12), des sous-pistons (13, 14) pour effectuer un premier déplacement, qui est effectué par les sous-pistons, différents du premier sous-piston (12), des sous-pistons (13, 14) par rapport au premier sous-piston (12), le premier déplacement étant nécessaire pour amener le piston télescopique (11) de l'état rentré dans l'état déployé.

2. Injecteur selon la revendication 1, au moins une des paires formant un mécanisme d'enclenchement (21, 24) pour la paire respective, les sous-pistons (12, 13, 14) de la paire étant couplés rigidement les uns aux autres dans la direction d'insertion (7) dans l'état déployé au moyen du mécanisme d'enclenchement (21, 24).

3. Injecteur selon la revendication 2, le mécanisme d'enclenchement (21, 24) étant formé par une partie faisant saillie (23, 26), qui est formée par un des sous-pistons (12, 13, 14) de la paire et qui est précontraint contre l'autre des sous-pistons (12, 13, 14) de la paire, et par un évidement (22, 25), qui est pratiqué dans l'autre des sous-pistons (12, 13, 14) de la paire, la partie faisant saillie (23, 26) venant en prise avec l'évidement (22, 25) dans l'état déployé.

4. Injecteur selon l'une des revendications 1 à 3, l'injecteur (1) pour au moins une des paires comportant un corps de blocage (40), qui est disposé de manière à pouvoir être déplacé et/ou pivoté et peut donc être déplacé et/ou pivoté depuis une première position, dans laquelle un des sous-pistons (12, 13, 14) de la paire peut être déplacé par rapport à l'autre des sous-pistons (12, 13, 14) de la paire dans la direction d'insertion (7), dans une deuxième position, dans laquelle l'un des sous-pistons (12, 13, 14) de la paire bute contre le corps de blocage (40), lorsque l'un des sous-pistons (12, 13, 14) de la paire est déplacé dans la direction d'insertion (7) par rapport à l'autre des sous-pistons (12, 13, 14) de la paire, et est ainsi mis au point pour entraîner ensuite l'autre des sous-pistons (12, 13, 14) de la paire dans la direction d'insertion (7).

5. Injecteur selon l'une des revendications 1 à 4, les sous-pistons, différents du premier sous-piston (12), des sous-pistons (13, 14) étant disposés à l'intérieur du premier sous-piston (12) dans l'état rentré.

6. Injecteur selon l'une des revendications 1 à 5, le corps (2) d'injecteur comportant une butée (16), contre laquelle le premier sous-piston (12) est mis au point pour venir en butée et qui limite ainsi un déplacement longitudinal du premier sous-piston (12) dans le sens inverse de la direction d'insertion (7).

7. Injecteur selon l'une des revendications 1 à 6, l'injecteur (1) comportant un moyen de précontrainte (31), qui est mis au point pour transporter le piston télescopique (11) de l'état rentré dans l'état déployé.

8. Injecteur selon l'une des revendications 1 à 7, l'injecteur (1) comportant la lentille intraoculaire (60), qui est disposée entre le premier sous-piston (12) et l'ouverture (8) de pointe dans la direction d'insertion (7).

9. Emballage avec l'injecteur (1) selon l'une des revendications 1 à 8, l'injecteur (1) étant disposé dans l'emballage (70) et le piston télescopique (11) se trouvant dans l'état rentré.
